Europäisches Patentamt

**European Patent Office**

Office européen des brevets

(19)

(11) Publication number: **0 171 174**
A2

(12) # EUROPEAN PATENT APPLICATION

(21) Application number: **85304754.6**

(22) Date of filing: **03.07.85**

(51) Int. Cl.⁴: **C 12 M 1/22**

(30) Priority: **06.08.84 US 637868**

(43) Date of publication of application: **12.02.86**
**Bulletin 86/7**

(84) Designated Contracting States: **DE FR GB NL**

(71) Applicant: **Gorman, James W., 1303 Riverwood Drive,**
**Jackson Mississippi 39211 (US)**

(72) Inventor: **Gorman, James W., 1303 Riverwood Drive,**
**Jackson Mississippi 39211 (US)**

(74) Representative: **Abbie, Andrew Kenneth et al, A.A.**
**THORNTON & CO. Northumberland House 303/306 High**
**Holborn, London, WC1V 7LE (GB)**

(54) **Petri dish which can be locked in different positions to admit ambient atmosphere at different rates.**

(57) The cover and bottom of a Petri dish have confronting walls equipped with abutments and positioning members. When those abutments and positioning members are moved out of register with each other, the cover can be readily assembled with or separated from that bottom; but, when those abutments and positioning members are in register, the cover will be locked against separation from the bottom. The positioning members have dwells and rises thereon which enable the cover to be locked in assembled relation with the bottom while permitting the ambient atmosphere to contact the contents of the dish at different, desired rates, thereby increasing the shelf life of those contents.

-1-

## PETRI DISH WHICH CAN BE
## LOCKED IN DIFFERENT POSITIONS
## TO ADMIT AMBIENT ATMOSPHERE AT DIFFERENT RATES

Summary of the Invention:

It is desirable to be able to lock the cover and bottom of a Petri dish against separation; and it can be desirable to permit the ambient atmosphere to contact the contents of that dish at different rates while that dish is locked. The present invention provides a cover, for a Petri dish, with a wall which can confront part of the wall of the bottom of that dish; and it also provides abutments and positioning members on those walls which can lock that cover on that bottom while permitting the ambient atmosphere to contact the contents of that dish at different rates. The preferred embodiment provides for such locking action with a limited amount of protrusion of the locking members, and minimizes the snagging effect of the locking mechanism.

Other advantages of the present invention should become apparent from an examination of the drawing and accompanying description.

In the drawing and accompanying description, a preferred embodiment of the present invention is shown and described, but it is to be understood that the drawing and accompanying description are for the purpose of illustration only and do not limit the invention and that the invention will be defined by the appended claims.

Brief Description of the Drawing:

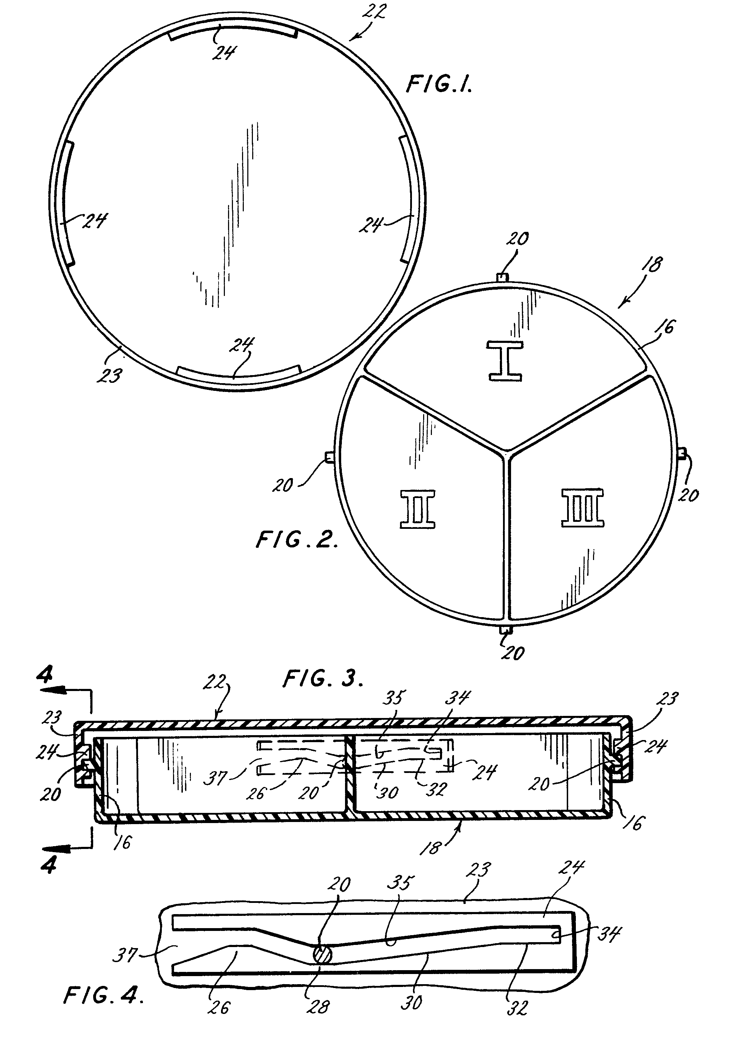

In the drawing, Fig. 1 is a bottom view of the cover of a Petri dish which is made in accordance with the principles and teachings of the present invention,

Fig. 2 is a plan view of the bottom of that Petri dish,

Fig. 3 is a vertical section, on a larger scale, through the cover and bottom of the Petri dish of Figs. 1 and 2, and

Fig. 4 is a sectional view, on a still larger scale, which is taken along a plane indicated by the line 4-4 in Fig. 3.

Detailed Description of the Preferred Embodiment:

The numeral 18 generally denotes the bottom of a Petri dish which has an upstanding wall 16 with four tab-like abutments 20 projecting outwardly from the outer face thereof. As shown by Fig. 2, the center of each of those abutments is spaced from the centers of adjacent abutments by about ninety degrees.

The numeral 22 generally denotes a cover for the Petri dish; and that cover has a wall 23 which can telescope downwardly over the upper portion of the wall 16. Four generally-rectangular positioning members 24 are formed on the inner surface of the wall 23; and each of those positioning members extends circumferentially of that inner surface. The center of each of those positioning members is spaced from the centers of adjacent positioning members by ninety degrees. Each of those positioning members has a surface that is displaced above the bottom thereof and that has a rise and fall which define a projection 26, a dwell 28, a rise 30, a dwell 32, and a stop 34. Also, each positioning member 24 has a many-faceted surface 35 that is substantially identical to, but spaced upwardly above, the surface which has the projection 26, the dwells 28 and 32 and the rise 30 thereon. The surface 35 is displaced below the upper surface of the positioning member 24. In this way, a barrel-cam track 37 is provided for each positioning member 24 and each such track is dimensioned to closely confine one of the abutments 20 while permitting ready movement of that abutment along that track. The entrance to each track is quite large to facilitate ready entry of an abutment 20.

As indicated particularly by Fig. 1, there are substantial circumferential gaps between the confronting ends of the various positioning members 24; and each of those gaps is much larger than the circumferential extent of any of the abutments 20. As a result, the wall 23 of the cover 22 can be telescoped downwardly over the upper portion of the wall 16 of the bottom 18, in a number of positions of that cover relative to that bottom, whenever the abutments 20 are out of register with all portions of the positioning members 24. At such times, the surfaces which define the lower portions of the tracks 20 of those positioning members can be set below the levels of the bottoms of the tab-like abutments 20 on the wall 16.

Thereafter, by rotating the cover 22 clockwise relative to the bottom 18, desired portions of the lower surfaces of the tracks 37 of the positioning members 24 can be moved into position beneath the abutments 20; and, at such time, those abutments and those portions of those tracks will lock the cover 22 against separation from the bottom 18. During the initial portion of the relative rotation between cover 22 and bottom 18, the abutments 20 will engage the rises of the projections 26, and hence will be guided over those projections. As the relative rotation of cover 22 and bottom 18 is continued, the abutments 20 will pass along the falls of the projections 26 and move into register with the dwells 28. If the relative rotation of cover 22 and bottom 18 is terminated at that time, the positioning portions of members 24 which define the dwells 28 will underlie the abutments 20 to lock that cover to that bottom, the projections 26 will prevent accidental relative rotation of that cover and bottom in the reverse or counter clockwise direction to un-locked position, and the portions of the surfaces 35 in register with the dwells 28 will hold the inner surface of cover 22 an appreciable distance above the upper edge of wall 16. As a result, the ambient atmosphere will be able to freely enter the dish at the gaps between the members 24 -- through the space between the walls 16 and 23 and then between the upper edge of wall 16 and the inner surface of cover 22 -- to contact the contents of that dish.

If it is desirable to reduce the rate at which the ambient atmosphere can enter the dish, the cover 22 can be rotated relative to the bottom 18 to dispose the abutments 20 in register with any desired points along the rises 30 on the positioning members 24. The closer those points are to the dwells 28, the further the surfaces 35 can hold the inner surface of cover 22 above the upper edge of wall 16 -- and hence the greater the rate at which the ambient atmosphere can enter the dish. The closer those points are to the dwells 32, the smaller the distances which the surfaces 35 can hold the inner surface of cover 22 above the upper edge of wall 16 -- and hence the

-4-

smaller the rate at which the ambient atmosphere can enter that dish. This means that by controlling the extent of relative rotation of the bottom 18 and cover 22, the user can select any desired rate at which the ambient atmosphere can pass through the circumferential gaps between the positioning members 24, can move upwardly through the annular space between the upper portions of the walls 16 and 23, and can then pass through the space between the upper edge of wall 16 and the inner surface of cover 22. The stops 34, at the ends of the dwells 32, enable the user of the covered Petri dish to know when the abutments 20 have reached the ends of the dwells 32.

If the user wishes to limit, as much as possible, the rate at which the ambient atmosphere can enter the dish, the abutments 20 will be moved into register with the dwells 32. At such time, the upper edge of the wall 16 will be held in tight abutting engagement with the inner surface of cover 22; and that wall and that cover will largely keep the ambient atmosphere from entering the dish.

In the preferred embodiment of the present invention, each of the positioning members 24 is thirty-two (32) millimeters long and three (3) millimeters high. The rise of the projection 26 has a vertical extent of one (1) millimeter and a horizontal extent of five (5) millimeters. A short dwell of one (1) millimeter in length is interposed between the rise and fall of the projection 26; and the vertical extent of that fall is one and one-half (1 1/2) millimeters and the horizontal extent of that fall is five (5) millimeters. That short dwell is spaced two (2) millimeters from the bottom of the positioning member 24. The dwell 28 has a horizontal extent of two (2) millimeters, and it is spaced one-half (1/2) of a millimeter from the bottom of the positioning member 24. The rise 30 has a vertical extent of one and one-half (1 1/2) millimeters, and it has a horizontal extent of twelve (12) millimeters. The upper end of that rise merges into the dwell 32; and that dwell has a horizontal extent of slightly less than seven (7) millimeters. The rise 30 is shallow enough so the abutments 20 will remain in any desired position in which they are set relative to that rise.

The radial dimension of each abutment 20 is less than one and one-half (1 1/2) millimeters; and the radial dimension of each positioning member 24 also is less than one and one-half (1 1/2) millimeters. As a result, the radial dimension of the annular space between the inner surface of the wall 23 of cover 22 and the outer surface of the wall 16 of bottom 18 can be as small as one and one-half (1 1/2) millimeters. Also, each abutment 20 will project such a short distance outwardly beyond the outer surface of wall 16 that the bottom 18 will not tend to snag nearby objects. The small size and the inward location of the positioning members 24 also alleviates snagging against other objects so that a snag obviating locking and fastening mechanism is provided.

In some instances it is desirable to maintain the moisture content of material that is held in a Petri dish while (a) precluding the entry of contaminants and (b) precluding the loss of any of the solid material in that dish. The present invention attains this desirable result by providing a cover 22 which is substantially imperforate and which is substantially inflexible and which can bear against a substantially unyieldable top edge for the wall 16. The undersurface of cover 22 and the top edge of wall 16 will be mold smooth, but neither of them will have a ground surface. This means that the said undersurface and top edge will necessarily and inherently define minute spaces through which vapor can pass even when that undersurface is gravity-pressed against that top edge. As a result, the present invention provides a Petri dish which can permit maintenance of the moisture content of material held within that dish while substantially precluding the entry of contaminants into the dish and also essentially precluding the loss of solid matter from that dish.

The small spaces, between the inner surface of cover 22 and the top edge of wall 16 can be decreased by creating forces which urge that inner surface into tight engagement with that top edge. Those forces can be supplied by the interactions between abutments 20 and positioning members 24 -- as those abutments and the dwells 32 of those positioning members cam the inner surface of cover 22 into intimate engagement with the top edge of wall 16, and thereby substantially reduce the sizes of the small spaces between that inner surface and that upper edge.

It should be noted that the configurations and dimensions of the projections 26, of the dwells 28 and 32, of the various rises, of the falls, and of the stops 34 of the positioning members can be changed as desired. Also, the bottom 18 and cover 22 can be made as large or as small as desired. Moreover, that dish can be made to have any desired number of compartments.

If desired, markings can be placed on the lower portions of the walls 16 and 23. Those markings could indicate when the abutments 20 were in register with the dwells 28, and also could indicate when those abutments were in register with various points along the rises 30. As a result, those markings would make it easy for the user of the dish to select desired

-7-

spacings between the upper edge of wall 16 and the inner surface of cover 22. If desired, the markings could indicate linear distances between the upper edge of wall 16 and the inner surface of cover 22, or they could indicate the rate at which the ambient atmosphere could pass between the upper edge of wall 16 and the inner surface of cover 22 under predetermined conditions of temperature and pressure.

In the preferred embodiment of the present invention, the positioning members 24 are formed on the inner surface of the wall 23 of cover 22, and the abutments 20 are formed on the exterior of wall 16 of the bottom 18. However, if desired, the abutments 20 could be formed on the wall 23 of cover 22, and the positioning members 24 could be formed on the wall 16 of bottom 18. In such event the positioning members 24 would be inverted from the position shown in Fig. 3; and the abutments 20 would underlie, rather than overlie, the projections 26, dwells 28 and 32, and rises 30 of those positioning members whenever the cover 22 was locked to the bottom 18.

In those instances where the users of Petri dishes want to be able to control the moisture content of material that is held in those dishes, but do not desire to hold the inner surface of the cover 22 above the level of the upper edge of the wall 16, the cover 22 can be made without the surfaces 35. In such event, the positioning members 24 and the abutments 20 can coact to lock the cover 22 on the bottom 18 while (a) permitting that cover to rest lightly on the upper edge of the wall 16 or (b) clamping the inner surface of that cover in tight engagement with that upper edge. When that inner surface is resting lightly on that upper edge, moisture will be able to escape, albeit at a relatively-slow rate, through small spaces between the confronting areas of that inner surface and of that upper edge. However, when that inner surface is clamped in tight engagement with that upper edge by the abutments 20 and the dwells 32, the escape of moisture will be minimal, thereby increasing the shelf life of the moisture in the dish.

Whereas the drawing and accompanying description have shown and described a preferred embodiment of the present invention, it should be apparent to those skilled in the art that various changes may be made in the form of the invention without affecting the scope thereof.

CLAIMS:

1. A cover and bottom for a Petri dish which can be locked against separation from each other while having confronting surfaces thereon spaced different distances from each other to permit the ambient atmosphere to contact the contents of said Petri dish at different rates and which comprises a wall on said bottom and a wall on said cover and interacting means on said bottom wall and on said cover wall which can lock said cover against separation from said bottom while permitting said confronting surfaces on said bottom and on said cover to be set at said different distances from each other to permit said ambient atmosphere to contact the contents of said dish at different rates.

2. A cover and bottom for a Petri dish as claimed in claim 1 wherein said interacting means include a circumferentially-directed member and a relatively-movable abutment.

3. A cover and bottom for a Petri dish as claimed in claim 1 wherein said wall of said cover is disposable in register with said wall on said bottom, wherein said interacting means include a circumferentially-directed member on one of said walls, wherein said interacting means include an abutment on the other of said walls, and wherein said abutment and said circumferentially-directed member are movable into and out of engagement.

4. A cover and bottom for a Petri dish as claimed in claim 1 wherein said wall of said cover is disposable in register with said wall on said bottom, and wherein a space between said walls can permit said ambient atmosphere to contact said contents of said Petri dish whenever said confronting surfaces on said bottom and on said cover are spaced apart.

-10-

5. A cover and bottom for a Petri dish as claimed in claim 1 wherein said interacting means include a circumferentially-directed member and an abutment, wherein said circumferentially-extending member and said abutment are movable out of register with each other to permit said cover to be assembled with and separated from said bottom, wherein said circumferentially-directed member and said abutment can be moved into register with each other to lock said cover against separation from said bottom, and wherein said abutment and said circumferentially-directed member can be set at different positions relative to each other while they are in register with each other to provide said different rates at which said ambient atmosphere can contact said contents of said Petri dish.

6. A cover and bottom for a Petri dish as claimed in claim 1 wherein said interacting means include a circumferentially-directed member and an abutment, wherein said circumferentially-extending member and said abutment are movable out of register with each other to permit said cover to be assembled with and separated from said bottom, wherein said circumferentially-directed member and said abutment can be moved into register with each other to lock said cover against separation from said bottom, wherein said abutment and said circumferentially-directed member can be set at different positions relative to each other while they are in register with each other to provide said different rates at which said ambient atmosphere can contact said contents of said Petri dish, and wherein a stop at one end of said circumferentially-directed member can intercept said abutment to limit relative rotation of said cover and bottom in one direction.

7. A cover and bottom for a Petri dish which can be locked against separation from each other while permitting ambient atmosphere to contact the contents of said dish at different rates and which comprises a wall on said bottom, a substantially-impermeable cover which has a wall that can be set in partial registry with said wall of said bottom, said wall of said bottom having an upper edge which is disposable at different distances from said cover to permit the ambient atmosphere to pass through the space between said walls of said bottom and of said cover and between the under surface of said cover and said upper edge of said wall of said bottom to contact the contents of said dish at different rates, and interacting means on said walls which can lock said cover against separation from said bottom while permitting said upper edge of said wall of said bottom to be disposed at said different distances from said cover.

8. A cover and bottom for a Petri dish as claimed in claim 7 wherein said interacting means include a circumferentially-directed member on one of said walls, wherein said interacting means include an abutment on the other of said walls, and wherein a space between said walls can permit said ambient atmosphere to contact said contents of said dish whenever said upper edge of said wall of said bottom and said cover are spaced apart.

9. A cover and bottom for a Petri dish as claimed in claim 7 wherein said interacting means include a circumferentially-directed member and an abutment, wherein said circumferentially-directed member has a barrel-cam track intermediate the upper and lower surfaces thereof, wherein said track has rises and dwells, wherein said circumferentially-directed member and said abutment are movable out of register with

-12-

each other to permit said cover to be assembled with and separated from said bottom, wherein said barrel-cam track of said circumferentially-directed member and said abutment can be moved into register with each other to lock said cover against separation from said bottom, wherein said abutment and said barrel-cam track of said circumferentially-directed member can be set at different positions relative to each other while they are in register with each other to enable said rises and dwells of said barrel-cam track to dispose said cover at different distances from said upper edge of said wall of said bottom to provide said different rates at which said ambient atmosphere can contact said contents of said dish, and wherein each of said abutment and said positioning member have relatively-small radial dimensions to permit said space between said walls of said bottom and of said cover to be relatively small and to minimize snagging of nearby objects by said wall of said bottom.

10. A cover and bottom for a Petri dish which can be locked against separation from each other while permitting moisture to escape from said Petri dish but while keeping contamination out of said Petri dish and which comprises a substantially-inflexible, substantially-imperforate cover, a wall on said bottom and a wall on said cover, said cover having the under surface thereof disposable in abutting engagement with the upper edge of said wall of said bottom, and interacting means on said bottom wall and on said cover wall which can lock said cover against separation from said bottom while permitting said confronting surfaces on said bottom and on said cover to abut in gravity-induced engagement or can lock said cover against separation from said bottom while clamping said under surface of said cover in intimate engagement with said upper edge of said wall of said bottom.

-13-

*22*

*24*

*24*          *24*

*24*

*23*

**FIG.I.**

*20*

*18*

*16*

I

*20*

II          III

*20*

*20*

**FIG.2.**

*20*

**4**

*22*          **FIG. 3.**          *35*     *34*          *23*

*23*                                                        *24*

*24*                                              *24*

*20*          *37*     *26*   *20*   *30*   *32*          *20*          *16*

*16*                              *18*

**4**

*20*     *35*     *23*          *24*

*37*                                        *34*

**FIG.4.**     *26*     *28*     *30*     *32*